# EUROPÄISCHE PATENTSCHRIFT

(11) **EP 0 996 640 B1**
(45) Veröffentlichungstag und Bekanntmachung des Hinweises auf die Patenterteilung: **24.10.2001**
(21) Anmeldenummer: 98938654.5
(22) Anmeldetag: 26.06.1998
(51) Int. Cl.: C08B 1/06, C08B 11/08

(54) **VERFAHREN ZUR HERSTELLUNG VON CELLULOSE-DERIVATEN**
METHOD FOR PRODUCING CELLULOSE DERIVATIVES
PROCEDE DE PRODUCTION DE DERIVES DE CELLULOSE

(30) Priorität: 09.07.1997 DE 19729323
(43) Veröffentlichungstag der Anmeldung: 03.05.2000
(73) Patentinhaber: Wolff Walsrode AG, 29655 Walsrode (DE)
(72) Erfinder: KOCH, Rainhardt, D-51065 Köln (DE); BERENDES, Frank, D-50937 Köln (DE); FOSTER, John, Narara, NSW 2250 (AU); RAST, Hans-Georg, D-51465 Bergisch Gladbach (DE); ENGELHARDT, Jürgen, D-51373 Leverkusen (DE); NEUBAUER, Jörg, D-29664 Walsrode (DE); KOCH, Wolfgang, D-29699 Bomlitz (DE); SZABLIKOWSKI, Klaus, D-29664 Walsrode (DE)
(74) Vertreter: Pettrich, Klaus-Günter, Dr.
(86) Internationale Anmeldenummer: EP9803907
(87) Internationale Veröffentlichungsnummer: WO9902568

(56) Entgegenhaltungen:
- WO-A-93/17174
- DE-C- 4 440 245
- PATENT ABSTRACTS OF JAPAN vol. 5, no. 45 (C-48), 25. März 1981 & JP 55 165901 A (BAIORISAAC CENTER KK), 24. Dezember 1980 & DATABASE WPI Week 8109 Derwent Publications Ltd., London, GB; AN 14804D
- PATENT ABSTRACTS OF JAPAN vol. 97, no. 7, 31. Juli 1997 & JP 09 065890 A (SHOWA DENKO KK), 11. März 1997 & CHEMICAL ABSTRACTS, vol. 126, no. 21, 26. Mai 1997 Columbus, Ohio, US; abstract no. 276439,
- J.PULS ET AL.: "Reaktionen isolierter Cellulasen, Hemicellulasen und Ligninasen an Faserstoffen und isolierten Holzkomponenten." PAPIER, DAS., Bd. 47, Nr. 12, 1993, Seiten 719-728, XP002081603 DARMSTADT DE
- KARL BREDERECK ET AL.: "Die Behandlung von Baumwolle und Cellulose-regeneratfasern mit Cellulase." MELLIAND TEXTILBERICHTE., Bd. 76, Nr. 9, 1995, Seiten 684-691, XP002081604 HEIDELBERG DE

## Beschreibung

Die vorliegende Erfindung betrifft ein Verfahren zur Herstellung von Cellulose-Derivaten unter Verwendung von mit Endoglucanasen vorbehandelter Cellulose.

Eine Reduktion der einzusetztenden Alkalimenge durch die Verwendung von Cellulasen in einer enzymatischen Vorbehandlungsstufe wurde von Michels und Meister beschrieben (DE 4 440 245 C1). Cellulasen sind Enzymkomplexe, in denen Enzyme mit unterschiedlichen katalytischen Aktivitäten vereinigt sind: Endoglucanasen (EC 3.2.1.4), Exoglucanasen, die auch als Cellobiohydrolasen bezeichnet werden (EC 3.2.1.74, EC 3.2.1.91) und β-Glucosidasen (EC 3.2.1.21). Diese Enzymaktivitäten gemeinsam bauen Cellulose vollständig zu Glucose ab, während jede für sich allein nur Teilschritte des Abbaus katalysiert. Endoglucanasen spalten z.B. nur endogene β-1,4-glykosidischen Bindungen in den amorphen Bereichen des Polymers.

Überraschenderweise können auch Endoglucanasen allein in einer ähnlichen Vorbehandlungsstufe zur Reduzierung des Alkalibedarfs verwendet werden. Darüber hinaus bietet die Verwendung von Endoglucanasen im Vergleich zu Cellulasen zwei wichtige Vorteile. Erstens führt die Vorbehandlung mit Cellulasen zu einem beträchtlichen Verlust an Cellulose-Substrat zu einer deutlichen Verringerung des Polymerisationsgrads (DP-Werts) und zu einem Verlust an Cellulose-Substrat. Zweitens wird die Cellulase-Aktivität durch lösliche oligomere Abbauprodukte gehemmt, was die Möglichkeit der wiederholten Verwendung einer Cellulase-Lösung erheblich einschränkt. Dieser Unterschied ist auf die unterschiedlichen katalytischen Aktivitäten der beiden Enzyme zurückführbar.

Die Erfindung betrifft somit ein Verfahren, mit dem Cellulose in technisch geeigneter Qualität vor der chemischen Umwandlung zu handelsüblichen Cellulose-Derivaten mit Endoglucanasen vorbehandelt wird. Mit Hilfe dieses Vorbehandlungsverfahrens kann der Alkalisierungsgrad der Cellulose und damit auch die Menge der in den Nachbehandlungsschritten eingesetzten Chemikalien beträchtlich verringert werden. Dieses Verfahren beinhaltet die enzymatische Behandlung der Cellulose mit Endoglucanasen vor Einbringung in den industriellen Herstellungsprozeß. Die auf diese Weise vorbehandelte und von der Enzymlösung getrennte Cellulose wird nachstehend als "aktiviert" bezeichnet. Die durch chemische Umwandlung von aktivierter Cellulose hergestellten Cellulose-Derivate sind vergleichbar mit den heutigen industriell hergestellten Produkten.

Erfindungsgemäß ist folgendes Verfahren:
(A) Eine bekannte Masse Cellulose wird durch Inkubation bei einer bestimmten Temperatur und über einen bestimmten Zeitraum in einem geeigneten Puffersystem mit Endoglucanase vorbehandelt.
(B) Die vorbehandelte Cellulose wird sodann von dem Vorbehandlungsgemisch aus Puffer und Enzym getrennt.
(C) Die "aktivierte" Cellulose wird anschließend wie unter industriellen Bedingungen chemisch umgesetzt, wobei jedoch erheblich weniger Alkali erforderlich ist.

Die Bedingungen für die enzymatische Vorbehandlung in Schritt (A) können nach Belieben variiert werden. Faktoren, die einen Einfluß auf die enzymatische Vorbehandlung haben, sind:
(a) die Herkunft der Endoglucanasen, die aus Pilzen, Bakterien und Pflanzen, bevorzugt aus den Pilzen Trichoderma reesei, Humicola insolens und Bakterien der Genera Bacillus, Cellulomonas, Sporocytophaga, Cytophaga, Clostridium stammen. Besonders bevorzugt ist die Verwendung von Denimax Ultra L® (Novo Nordisk)
(b) Pufferkonzentration von 1 bis 1000 mM , bevorzugter Bereich 10 bis 100 mM, besonders bevorzugter Bereich 50 mM Natriumacetat bzw. Kaliumphosphat je nach erforderlichem Pufferbereich. Erfindungsgemäß sind auch andere Puffer oder Puffer-Lösungsmittelgemische.
(c) pH-Wert des Puffers im Bereich pH 1 bis pH 13, bevorzugter Bereich pH 4 bis pH 10, besonders bevorzugter Bereich pH 5 bis pH 7,5.
(d) Verhältnis von Cellulose-Masse zu Puffervolumen zwischen 1 g zu 0,5 ml und 1 g zu 1000 ml, bevorzugt zwischen 1 g zu 5 ml und 1 g zu 100 ml, besonders bevorzugt zwischen 1 g zu 10 ml und 1 g zu 30 ml.
(e) Verhältnis von Enzymmasse zu Cellulosemasse 0,01 bis 50 %, bevorzugter Bereich 1 bis 30 % besonders bevorzugter Bereich 3 bis 12 %.
(f) Inkubationstemperatur von 0 bis 100°C, bevorzugter Bereich 20 bis 80°C, besonders bevorzugter Bereich 50 bis 60°C.
(g) Inkubationszeit von wenigen Minuten bis zu mehreren Tagen, bevorzugter Bereich 1 bis 24 Stunden, besonders bevorzugter Bereich 2 bis 3 Stunden.
(h) Schütteln bzw. Rühren des Inkubationsgemischs mit 1 bis 10.000 UpM, bevorzugter Bereich 10 bis 2000 UpM, besonders bevorzugter Bereich 200 bis 300 UpM.

Die Beeinflussung der Enzymwirkung durch diese Faktoren ist an sich bekannt. Darüber hinaus ist nachgewiesen, daß die aus verschiedenen Arten gewonnenen cellulolytischen Enzyme unterschiedliche Affinitäts- und Aktivitätsgrade aufweisen. Die Änderung dieser Faktoren im Vorbehandlungsverfahren wird daher Einfluß auf die Aktivierung der Cellulose haben. Dies führt wiederum zu Änderungen im Substitutionsgrad der chemisch umgesetzten Cellulose-Derivate.

Die Auswirkungen der enzymatischen Vorbehandlung auf die Substitutionswerte von chemisch umgesetzter Cellulose können auf zweierlei Weise gezeigt werden. Erstens weist die enzymatisch vorbehandelte Cellulose gegenüber unbehandelten Kontrollen signifikant höhere Werte für die Molekularsubstitution (MS) auf. Zweitens war die Menge des für die chemischen Umwandlungsreaktionen erforderlichen Alkalis bei Verwendung von vorbehandelter Cellulose signifikant geringer. Die Erfindung hat daher den Vorteil, die für den Umwandlungsprozeß erforderliche Alkalimenge zu reduzieren und dennoch das gleiche Endprodukt zu liefern. Darüber hinaus bietet die Verwendung von Endoglucanasen in der Vorbehandlungsstufe gegenüber Cellulasen zwei wichtige Vorteile:

In den Abbildungen werden folgende Zusammenhänge gezeigt:

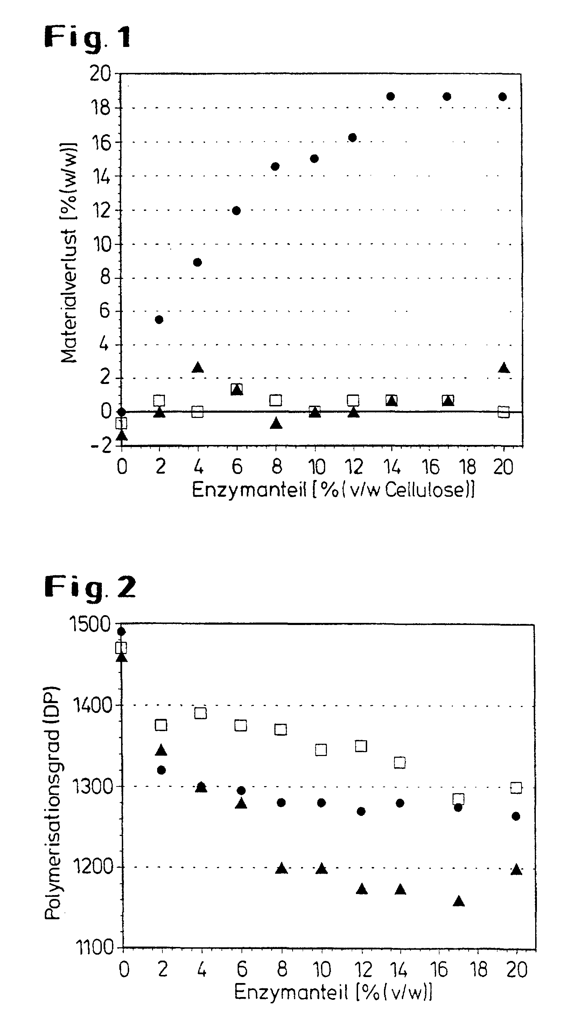

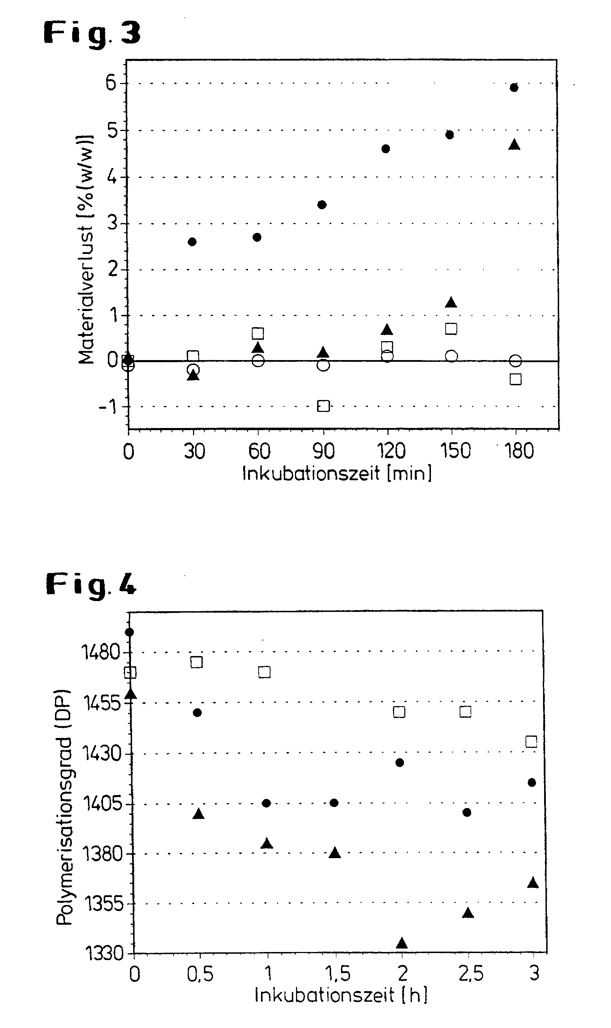

Die Vorbehandlung mit Cellulase® führte zu einem signifikanten Materialverlust, der an einer Gewichtsabnahme und an der Veringerung des DP-Werts zu erkennen war. Die untersuchten Endoglucanasen zeigten hingegen nur einen vernachlässigbaren Verlust an Cellulose (Abb. 1 und 3). Der Materialverlust war auf den Abbau der Cellulose zu löslichen Monomer- und Oligomerprodukten in Schritt (A), einen Verlust an Feinstpartikeln bei der Trennung in Schritt (B) und einen Verlust an Material bei der Überführung in die chemischen Reaktionsgefäße bei (B/C) zurückführen. Nach 20 Stunden Inkubation bei einer Temperatur von 36°C und einem Puffer-pH von 5 zeigte die untersuchte Cellulose je nach Enzymkonzentration einen allmählichen Materialverlust von bis zu 19 % des Ausgangsgewichts.

Im Gegensatz dazu verursachten die Endoglucanasen einen vernachlässigbaren Gewichtsverlust (Abb. 1). Die Inkubation von Cellulose mit einer Cellulase® -Konzentration von 2 % (w/w Enzym- zu Cellulosegewicht) bei optimalen Bedingungen, d.h. pH 5,5 und 50°C, ergab ebenfalls einen allmählichen Materialverlust von etwa 6 % nach 180 Minuten Inkubationszeit. Cellulose-Proben mit einer Konzentration von 6 % bzw. 15 % (v/w - Enzymvolumen zu Cellulosegewicht) der Endoglucanase Denimax Ultra L® zeigten nach der gleichen Inkubationszeit bei einem optimalen pH von 7 und einer Temperatur von 60°C einen vernachlässigbaren Materialverlust. Eine Konzentration von 6 % Cellusoft Ultra L® ergab nach 90 Minuten bei pH 5,5 und 50°C einen vernachlässigbaren Materialverlust; danach kam es zu einem fast exponentiellen Verlust an Cellulosematerial bis zu etwa 5 % nach 180 Minuten (Abb. 3).

Die Cellulase-Aktivität wird durch die löslichen oligomeren Abbauprodukte gehemmt. Folglich ist die Verwendung des Puffer-/Cellulase-Gemischs nach Trennung der vorbehandelten Cellulose in Schritt (B) eingeschränkt. Das Fehlen von gelösten Abbauprodukten und ihrer Hemmwirkung auf die Endoglucanasen bedeutet, daß das Puffer-/Endoglucanase-Gemisch ohne weiteres zur Vorbehandlung weiterer Cellulose-Proben wiederverwendet werden kann, wodurch die Materialkosten gesenkt werden. Neben dem Nachweis der Abbauaktivität der in den nachstehenden Beispielen verwendeten Enzyme sind diese Kurven auch wesentlich für die Berechnung von normalisierten vorbehandelten Probengewichte. Auf diese Weise lagen nach der Vorbehandlung die gleichen äquivalenten Trockengewichte an Cellulose-Material für jede Probe vor und wurden den gleichen chemischen Umwandlungsbedingungen ausgesetzt.

Abb. 2 zeigt die Veränderungen im Polymerisationsgrad (DP) für mit verschiedenen Konzentrationen von Cellulase und Endoglucanasen gemäß der Beschreibung zu Abb. 1 vorbehandelte Cellulose-Proben. Der Polymerisationsgrad nahm mit zunehmender Enzymkonzentration ab, so daß maximale Unterschiede von etwa 13 % und 15 % für mit Denimax Ultra L® bzw. Cellulase® vorbehandelte Proben ermittelt wurden. Die Cellusoft Ultra L® -Proben zeigten eine etwas höhere Endo-Aktivität mit einer 20 %igen Verringerung gegenüber den Proben ohne enzymatische Vorbehandlung. Ein ähnlicher Trend wurde für die unter optimalen Bedingungen vorbehandelten Proben beobachtet (Abb. 4). In diesen Fällen fiel die Abnahme des Polymerisationsgrades mit jeweils weniger als 10 % nicht geringer aus.

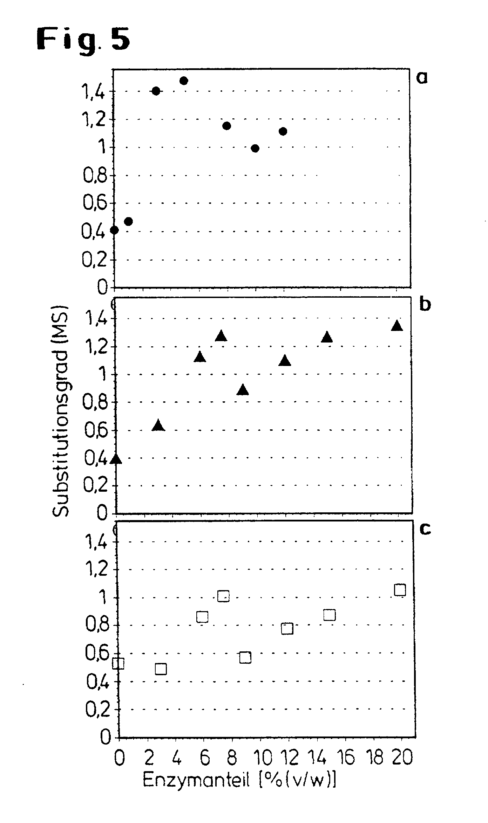

Die enzymatische Vorbehandlung der Cellulose hatte eine deutliche Auswirkung auf den Substitutionsgrad der Cellulosederivate, die mit Propylenoxid im alkalischen Milieu zu Hydroxypropylcellulose (HPC) verethert wurden (Abb. 5). Während eine Inkubation nur in Puffer (Enzymanteil = 0 %) zu Substitutionsgraden zwischen 0,4 und 0,5 Mol-% erreicht werden. Dabei zeigten die Endoglucanasen (Denimax Ultra L® und Cellusoft Ultra L® ) die gleiche Wirksamkeit wie die Cellulase. Die Veränderung der Substitution in Abhängigkeit von der Enzymkonzentration war bei allen getesteten Enzymen durch das Auftreten von Optima gekennzeichnet.

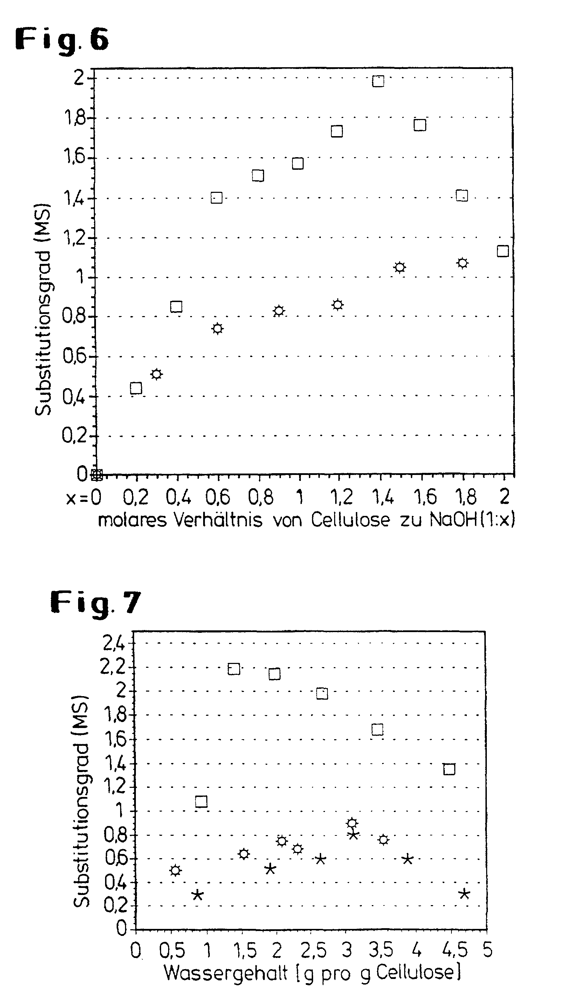

Abb. 6 zeigt die Auswirkungen von Alkali auf die Substitutionswerte der Cellulose-derivate nach der chemischen Umsetzung. Steigende Mengen NaOH ergaben bei nur mit Puffer vorbehandelter Cellulose maximale MS-Werte von 1,1 Mol-%. Durch eine Vorbehandlung mit der Endoglucanase Denimax ultra L konnten MS-Werte von bis zu 2Mol-% erreicht werden. Am deutlichsten trat der Effekt der enzymatischen Akitiverung nach einer chemischen Umsetzung in Gegenwart von 1,2 bis 1,6 g NaOH pro g Cellulose hervor. Denimax Ultra L wurde mit 15 % v/w eingesetzt und die Umsetzung erfolgte bei 200 UpM. Unter diesen Bedingungen wurden zur Herstellung von HPC mit einen Substitutionsgrad von 1,0 nach enzymatischer Vorbehandlung ca. 28 % weniger NaOH benötigt.

Der Substitutionsgrad kann bei den Proben mit enzymatisch vorbehandelter Cellulose als Substrat durch eine Verringerung des Wassergehalts im Reaktionsgemisch noch weiter gesteigert werden. Cellulose-Proben wurden durch Inkubieren über 2 Stunden bei 60°C unter starkem Schütteln (200 UpM) in Wasser, Puffer und Puffer mit Enzym vorbehandelt (Abb. 7). Nach dem Inkubieren wurden die Proben unter leichtem Druck filtriert. Die Wasserretentionswerte für die Cellulose-Proben variierten je nach Filtrationsdauer und -druck. Die relativen Wasserretentionswerte zwischen den verschiedenen Behandlungsverfahren blieben jedoch gleich. Dies zeigt, daß bei den hier gewählten Versuchsbedingungen die enzymatische Vorbehandlung zwar die Cellulosestruktur ändert, jedoch keine signifikante Änderung des Wasserrückhaltevermögens bewirkt. Die chemische Umwandlung dieser so vorbehandelten Proben zeigte jedoch deutliche Unterschiede im molaren Substitutionsgrad ihrer Derivate. Die aus enzymatisch vorbehandelter Cellulose hergestellten Derivate wiesen bis zu 91% höhere Substitutionsgrade gegenüber nicht enzymatisch behandelter Cellulose-Proben auf. Durch Optimierung des Wassergehalts im Reaktionsgemisch konnte diese Erhöhung signifikant auf 161 % gesteigert werden.

Abb. 7 zeigt auch den Einfluß des an der Reaktion beteiligten Wassers auf die Derivatsynthese. Bei durch Inkubation in Wasser vorbehandelten Cellulose-Proben wiesen die synthetisierten Derivate eine maximale Molekularsubstitution von 0,8 mit einem Wassergehalt von 3,1 g pro 1g Cellulose auf Mit Puffer vorbehandelte Proben besaßen ein ähnliches Wassergehalts-Optimum mit einer geringfügig höheren Molekularsubstitution von etwa 0,88. Mit abnehmendem Wassergehalt zeigten die mit Puffer vorbehandelten Proben zunehmend höhere MS-Werte als mit Wasser behandelte Proben. Im Gegensatz dazu wiesen aus enzymatisch vorbehandelten Proben synthetisierte Derivate bei allen untersuchten Wassergehalten höhere MS-Werte auf, mit einem maximalen MS-Wert von 2,35 bei 1,45 g Wasser pro 1g Cellulose. Somit erforderten die als Substrate für die chemische Umwandlung verwendeten enzymatisch vorbehandelten Cellulose-Proben etwa 47 % weniger Wasser, um gegenüber nicht enzymatisch behandelten Proben einen um 161 % höheren maximalen MS-Wert zu erreichen.

### Beschreibung der Abbildungen

- **Abb. 1**: **Materialverlust an Cellulose durch Einwirkung handelsüblicher cellulolytischer Enzyme.** Cellulase® von Merck (●), Endoglucanasen - Denimax Ultra L® (□ ) und Cellusoft Ultra L® (∇) von Novo Nordisk. 1,5 g-Proben Linters Temming T 500 wurden bei 36°C geschüttelt (200 UpM) und 20 Stunden lang mit verschiedenen Enzymkonzentrationen inkubiert. Die Cellulase® - und Cellusoft Ultra L® -Proben wurden in 50 mM Natriumacetatpuffer mit einem pH-Wert von 5 und die Denimax Ultra L® -Proben in 50 mM Kaliumphosphatpuffer mit einem pH-Wert von 7,1 inkubiert. Nach der Inkubation wurden die Proben in einem Eisbad gekühlt, filtriert und unter leichtem Druck gewaschen. Die Naßgewichte des vorbehandelten Cellulosematerials wurden gemessen und die Proben in einem Vakuumofen bei 65°C über 24 Stunden getrocknet. Vor der Bestimmung der Trockengewichte wurden die Proben auf Raumtemperaturabgekühlt. Die Materialverluste wurden als Prozentsatz der Ausgangsgewichte angegeben.
- **Abb. 2**: **Veränderung des Polymerisationsgrads der Cellulose durch Einwirkung handelsüblicher cellulolytischer Enzyme.** Cellulose-Proben wurden in Gegenwart verschiedener Konzentrationen der Enzyme Cellulase® (●), Denimax Ultra L® (□ ) und Cellusoft Ultra L® (∇) gemäß Abb. 1 abgebaut, analysiert und es wurden ihre jeweiligen Polymerisationsgrade bestimmt.
- **Abb. 3**: **Zeitlicher Verlauf des Materialverlusts an Cellulose.** Cellulase® (●), Denimax Ultra L® (□ und ○) und Cellusoft Ultra L® (∇). 1,5 g-Proben Baumwollcellulose von Wolff Walsrode wurden bei 50°C geschüttelt (200 UpM) und mit 2 % (w/w, Enzym- zu Cellulosegewicht) Cellulase® in 50 mM Natriumacetatpuffer mit einem pH-Wert von 5,5 inkubiert. 1,5 g-Cellulose-Proben wurden unter gleichen Bedingungen mit 6 % (v/w Enzymvolumen zu Cellulosegewicht) Cellusoft Ultra L® inkubiert. Weitere 1,5 g-Cellulose-Proben wurden bei 60°C geschüttelt (200 UpM) mit 6 % (□ ) bzw. 15 % (v/w) (O) Denimax Ultra L® in 50 mM Kaliumphosphatpuffer mit einem pH-Wert von 7,0 inkubiert. Die Proben wurden in regelmäßigen zeitlichen Abständen entnommen und ihre Naß- und Trockengewichte gemäß Abb. 1 bestimmt. Die Materialverluste wurden als Prozentsatz der Ausgangsgewichte angegeben.
- **Abb. 4**: **Zeitlicher Verlauf der Änderung des Polymerisationsgrads der Cellulose.** Cellulose-Proben wurden in Gegenwart verschiedener Konzentrationen der Enzyme Cellulase® (●), Denimax Ultra L® (□ ) und Cellusoft Ultra L® (∇) gemäß Abb. 3 abgebaut, analysiert und es wurden ihre jeweiligen Polymerisationsgrade bestimmt.
- **Abb. 5**: **Änderung des Substitutionsgrads enzymatisch vorbehandelter Cellulose-Derivate in Abhängigkeit der Enzymkonzentration**. Die Cellulose-Proben wurden vor der chemischen Umwandlung mit Puffer und verschiedenen Enzymkonzentrationen vorbehandelt. Die Enzymvorbehandlung der Proben mit (**a**) Cellulase® (●), (**b**) Cellusoft Ultra L® (∇) und (**c**) Denimax Ultra L® (□ ) erfolgte gemäß der Beschreibung zu Abb. 1. Vorbehandelte Cellulose-Proben mit einem Gewicht von je 5 g und mit Wasserretentionswerten von 2 g/g wurden sodann durch Inkubation bei 80°C unter Schütteln bei 50 UpM über 3 Stunden in Anwesenheit von 50 ml eines Gemischs aus Dioxan und Wasser (9:1) bei einem Molverhältnis von Cellulose zu Propylenoxid von 1:5 und einem Molverhältnis von Cellulose zu 50 % Natriumhydroxid von 1:1,5 chemisch umgesetzt. Der Substitutionsgrad des Produkts wurde mittels Festkörper-NMR bestimmt.
- **Abb. 6**: **Änderung des Substitutionsgrads enzymatisch vorbehandelter Cellulose-Derivate in Abhängigkeit des Alkalikonzentration.** Cellulose-Proben wurden mit Kaliumphosphatpuffer ( 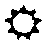 ) bzw. einer Konzentration von 15 % (v/w) Denimax Ultra L® (□ ) gemäß der Beschreibung zu Abb. 2 vorbehandelt. Die vorbehandelten Proben mit einem Gewicht von je 5 g und mit Wasserretentionswerten von 2 g/g wurden gemäß der Beschreibung zu Abb. 5 chemisch umgesetzt, mit der Ausnahme, daß vor und während der chemischen Umwandlung mit 200 UpM geschüttelt wurde.
- **Abb. 7**: **Maximierung der Substitution enzymatisch vorbehandelter Cellulose-Derivate durch Optimierung des Wassergehalts**. Durch Vorquellung in Wasser (*) vorbehandelte Cellulose, mit Puffer ( 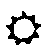 ) vorbehandelte Cellulose und mit Denimax Ultra L® (□ ) von Novo Nordisk vorbehandelte Cellulose. 5 g-Proben Linters Temming 500 wurden bei 60°C geschüttelt (200 UpM) und 2 Stunden lang in Kaliumphosphatpuffer (50 mM, pH 7) mit und ohne 6 % (v/w - Enzymvolumen zu Cellulosegewicht) Denimax Ultra L® Endoglucanase inkubiert. Mit Wasser vorbehandelte Proben wurden unter den gleichen Bedingungen nur in Wasser inkubiert. Nach der Inkubation wurden die Cellulose-Proben von dem Gemisch getrennt und der chemischen Umwandlung gemäß der Beschreibung Abb. 5 unterzogen.

### Beispiele

### Beispiel 1

### Enzymatische Vorbehandlung - Änderung der Enzymkonzentration.

Die in Abb. 1 gezeigten Abbaukurven wurden verwendet, um die Ausgangsgewichte der zur Herstellung von 5 g vorbehandeltem aktiviertem Cellulose-Substrat erforderlichen Cellulose zu berechnen. Proben technischer Baumwollcellulose mit einer hohen Kristallinität (> 80 %) und einem Polymerisationsgrad von etwa 1500 wurden bei 36°C unter verstärktem Schütteln (200 UpM) 20 Stunden lang in einem geeigneten Puffer mit einem Verhältnis von Cellulose zu Puffer von 1 g zu 15 ml in Anwesenheit von verschiedenen Enzymkonzentrationen inkubiert. Die Proben wurden in 50 mM Natriumacetatpuffer bei pH 5 mit Cellusoft Ultra L® -Konzentrationen von 0 bis 15 % (v/w) inkubiert. Proben mit gleichen Konzentrationen von Denimax Ultra L® wurden in 50 mM Kaliumphosphatpuffer bei pH 7,1 inkubiert. Nach der Inkubation wurden die Proben in einem Eisbad abgekühlt, filtriert und unter leichtem Druck mit einem Büchner-Trichter Nr. 4 gewaschen. Die vorbehandelten Proben mit äquivalenten Trockengewichten von 5 g und Wasserretentionswerten von 2 g/g Cellulose wurden sodann in Glasreaktionsgefäße überführt und dem in nachstehendem Beispiel 2 beschriebenen chemischen Umwandlungsverfahren unterzogen. Alle Stufen des Vorbehandlungsverfahrens wurden gravimetrisch untersucht und zeigten eine vollständige Überführung des Materials in die chemische Umwandlungsstufe.

### Beispiel 2

### Herstellung von Hydroxypropylcetlulose (HPC).

50 ml einer Lösung aus Dioxan und Wasser (9:1) wurden zu den mit Endoglucanase vorbehandelten Cellulose-Proben aus Beispiel 1 zugegeben. Diesen Mischungen wurden 50 %iges Natriumhydroxid und 100 %iges Propylenoxid in Molverhältnissen von 1:1,5 bzw. 1:5 für Cellulose zu Substanz zugesetzt und der Inhalt durch leichtes Schwenken vermischt. Sodann wurden die Proben unter Druck durch Reaktion bei 80°C über 3 Stunden unter leichtem Schütteln (50 UpM) umgesetzt. Die umgesetzten Proben wurden herausgenommen und 5 Minuten lang abkühlen gelassen. Das katalytische Alkali wurde durch Zugabe von 100 % Essigsäure in einem Molverhältnis von 1:1 neutralisiert. Die flüchtigen Inhaltsstoffe wurden durch Abstellen der Reaktionsgefäße in einem leichten Luftstrom in einem Abzug über 15 Stunden entfernt. Danach wurde die HPC durch starkes Mischen mit 200 ml destilliertem Wasser und Dialyse (MWCO 1000) des Gemisches über 5 Stunden unter fließendem destilliertem Wasser und anschließend über 15 Stunden in einem 2,5 Liter fassendem Wasserbad destilliertem Wasser bei 4°C gereinigt. Die durch Dialyse gereinigten Proben wurden sodann in Abdampfschalen unter staubfreien Bedingungen bei 70°C in einem kontinuierlichen Niederdruckstrom getrocknet. Die getrockneten HPC-Proben wurden anschließend durch Zermahlen bei tiefen Temperaturen zerkleinert und mittels Festkörper-NMR analysiert. Alle Stufen des Verfahrens wurden gravimetrisch untersucht und zeigten eine vollständige Überführung des Materials in die jeweils nachfolgende Verfahrensstufe. Die NMR-Ergebnisse wurden quantitativ zur Berechnung der Molekularsubstitution (MS) und qualitativ zur Bestätigung der Reinheit der HPC verwendet. Die Ergebnisse sind in Abb. 5 gezeigt und nachstehend zusammengefaßt.

| Probe | Opt.Konz. (%) | Mat.-verl. (%) | DP | DP (%) | MS | MS (%) |
|---|---|---|---|---|---|---|
| Natriumacetatpuffer, pH 5 | - | 0 | 1475 | 100 | 0,41 | 100 |
| Kaliumphosphatpuffer, pH 7 | - | 0 | 1470 | 100 | 0,50 | 100 |
| | | | | | | |
| Cellulase® | 4,0 | 9 | 1300 | 88,1 | 1,50 | 366 |
| Endoglucanase-Cellusoft Ultra L® | 6,6 | 0 | 1220 | 82,7 | 1,32 | 322 |
| Endoglucanase - Denimax Ultra L® | 7,0 | 0 | 1360 | 92,5 | 1,05 | 201 |

### Vergleichsbeispiele

Für die Beispiele 1 und 2 wurden nur mit Puffer und ohne Enzym vorbehandelte Kontrollen verwendet. Darüber hinaus wurde in einem Vergleich auch eine kommerziell erhältliche Cellulase der Firma Merck untersucht. In diesem Fall wurden die Cellulose-Proben durch Inkubation bei 36°C und 200 UpM über 20 Stunden in 50 mM Natriumacetatpuffer (pH 5) mit Cellulase® -Konzentrationen von 0 bis 15 % (w/w) vorbehandelt.

### Beispiel 3

### Enzymatische Vorbehandlung - Optimale Bedingungen.

Die in Abb. 3 gezeigten Abbaukurven wurden verwendet, um die erforderlichen Ausgangsgewichte zur Herstellung von vorbehandelten aktivierten Cellulose-Proben mit äquivalenten Trockengewichten von 5 g zu berechnen. Proben technischer Baumwollcellulose mit einer hohen Kristallinität (>80 %) und einem Polymerisationsgrad von etwa 1500 wurden bei 50°C unter starkem Schütteln (200 UpM) 2 Stunden lang in 50 mM Natriumacetatpuffer bei pH 5,5 mit einem Verhältnis von Cellulose zu Puffer von 1 g zu 15 ml in Anwesenheit einer Konzentration von 6 % (v/w) Cellusoft Ultra L® inkubiert. Proben mit Konzentrationen von 6 % und 15 % (v/w) Denimax Ultra L® wurden bei 60°C und 200 UpM über 2 Stunden in 50 mM Kalium phosphatpuffer bei pH 7,0 inkubiert. Nach der Inkubation wurden die Proben in einem Eisbad abgekühlt, filtriert und unter leichtem Druck mit einem Büchner-Trichter Nr. 4 gewaschen. Die vorbehandelten Proben mit äquivalenten Trockengewichten von 5 g und Wasserretentionswerten von etwa 2 g/g wurden anschließend dem in den nachstehenden Beispielen 4 und 5 beschriebenen chemischen Umwandlungsverfahren unterzogen. Alle Stufen des Vorbehandlungsverfahrens wurden gravimetrisch untersucht und zeigten eine vollständige Überführung des Materials in die chemische Umwandlungsstufe.

### Beispiel 4

### Herstellung von Hydroxypropylcellulose (HPC).

Die mit 6 % (v/w) Denimax Ultra L® vorbehandelte Cellulose aus Beispiel 3 wurde dem in Beispiel 2 beschriebenen chemischen Umwandlungsverfahren unterzogen, jedoch mit einer wichtigen Ausnahme: 50 % Natriumhydroxid wurde in variablen Molverhältnissen für Cellulose zu Alkali von 1:0 bis 1:2,0 zugegeben. Danach wurde die HPC gemäß Beispiel 2 gereinigt und mittels NMR untersucht. Die Ergebnisse sind in Abb. 6 gezeigt.

### Beispiel 5

### Herstellung von Hydroxypropylcellulose (HPC).

Die mit 15 % (v/w) Denimax Ultra L® vorbehandelte Cellulose aus Beispiel 3 wurde dem in Beispiel 4 beschriebenen chemischen Umwandlungsverfahren unterzogen, jedoch mit einer wichtigen Ausnahme: Die Schüttelgeschwindigkeit während der chem. Umwandlung wurde auf 200 UpM erhöht. Danach wurde die HPC gemäß Beispiel 2 gereinigt und mittels NMR untersucht. Die Ergebnisse sind in Abb. 7 gezeigt.

Für die Beispiele 3, 4 und 5 wurden nur mit Puffer und ohne Enzym vorbehandelte Kontrollen verwendet. Darüber hinaus lieferte ein Molverhältnis von 1:0 für Cellulose zu Natriumhydroxid bei der chemischen Umwandlung Kontrollen für die erforderliche katalytische Alkalimenge.

### Beispiel 6

5 g-Proben technischer Baumwollcellulose (>80 %, Polymerisationsgrad 1600) von Wolff Walsrode wurden bei 60°C unter verstärktem Schütteln (200 UpM) 2 Stunden lang in Puffer (pH 7, 50 mM) und in Puffer mit 15 % (vlw - Enzymvolumen zu Cellulosegewicht) der Endoglucanase Denimax Ultra L® (Novo Nordisk) inkubiert. Nach der Inkubation wurden die Cellulose-Proben in einem Eisbad abgekühlt und unter leichtem Druck filtriert. Anschließend wurden die Naßgewichte der Proben gemessen und die Wasserretentionswerte berechnet.

Sodann wurden die Proben durch Reaktion bei 80°C über 3 Stunden unter Schütteln bei 200 UpM in Anwesenheit von 30 ml eines Gemischs aus Dioxan und Wasser (9:1) zusammen mit Propylenoxid und Natriumhydroxid (50 %) in Molverhältnissen von Cellulose zu Reaktionspartner von 1:5 bzw. 1:1.5 zu Hydroxypropylcellulose (HPC) umgesetzt. Nach der Reaktion und anschließendem Abkühlen wurden die Proben durch Mischen mit Essigsäure (100 %) in einem Molverhältnis von Säure zu Alkali 1:1 neutralisiert. Die verdampfenden Reaktionspartner wurden durch Abstellen in einem leichten Luftstrom über etwa 15 Stunden entfernt. Danach folgte ein Reinigen der HPC durch Zumischen von 200 ml destilliertem Wasser und anschließende Dialyse (Dialyseschläuche MWCO 1000, Fa. Serva), zuerst mit einem kontinuierlichen Wasserstrom über 5 Stunden und dann in 5 Liter über 16 Stunden bei 4°C. Die gereinigten Proben wurden in Abdampfschalen unter reduziertem Druck bei 70°C über 20 Stunden getrocknet. Anschließend wurden die HPC-Proben durch Zermahlen bei tiefen Temperaturen zerkleinert, ehe die MS-Werte durch Festkörper-NMR bestimmt wurden. Alle Stufen des Verfahrens wurden gravimetrisch untersucht und zeigten eine vollständige Überführung des Materials in die jeweils nachfolgende Verfahrensstufe. Die Ergebnisse sind in der nachstehenden Tabelle angegeben.

### Beispiel 7

Cellulose-Proben wurden durch Inkubieren in Wasser, Puffer oder Puffer mit 6 % (v/w) Denimax Ultra L® gemäß Beschreibung in Beispiel 6 vorbehandelt. Vor der chemischen Umwandlung wurde den Reaktionsgemischen destilliertes Wasser in unterschiedlichen Mengen zugesetzt. Danach wurde HPC gemäß Beispiel 6 hergestellt, gereinigt und analysiert. Die Ergebnisse sind in der nachstehenden Tabelle und in Abb. 7 gezeigt.

| Veränderung der Bedingungen | Puffer | 6 % Denimax Ultra L® | 15 % Denimax Ultra L® | Zunahme | Beispiel |
|---|---|---|---|---|---|
| | MS-Werte | | | | |
| 1) Chemische Umwandlung unter Schütteln mit 200 UpM 2) Wassergehalt 1,7 g/g Cellulose | 0,93 | - | 1,79 | 91 % | 2 |
| 1) Chemische Umwandlung unter Schütteln mit 200 UpM Optimaler Wassergehalt | 0,90 | 2,35 | - | 161 % | 3 |

## Patentansprüche

1. Verfahren zur Herstellung von Hydroxyalkylcellulose-Ethern aus der Reaktion von Alkenoxiden und aktivierter Cellulose, **dadurch gekennzeichnet, dass** die Cellulose wie folgt vorbehandelt wird:
a) Inkubation in einer Pufferlösung oder Wasser oder einem Lösungsmittel-Puffer- bzw. Wassergemisch und Endoglucanase,
b) Trennung der mit Endoglucanase vorbehandelten Cellulose von dem Puffer- oder Wasser- oder Lösungsmittel-Puffer- bzw. Wassergemisch,
c) Umsetzung der aktivierten Cellulose zu substituierten Cellulose-Derivaten durch Reaktion mit Alkenoxiden in Anwesenheit von katalytischem Alkali.

2. Verfahren gemäß Anspruch 1, **dadurch gekennzeichnet, dass** man Endoglucanase aus Pilzen, Bakterien und Pflanzen verwendet.

3. Verfahren nach Anspruch 2, **dadurch gekennzeichnet, dass** man Endoglucanase aus den Pilzen Trichoderma reesei, Humicola insolens oder Bakterien der Genera Bacillus, Cellulomonas, Sporo-cytophaga, Clostridium oder Denimax Ultra L® verwendet.

4. Verfahren gemäß einem der Ansprüche 1 bis 3, **dadurch gekennzeichnet, dass** die Inkubation bei einer Temperatur zwischen 0°C und 100°C über 0,1 bis 24 Stunden durchgeführt wird.

5. Verfahren gemäß einem der Ansprüche 1 bis 4, **dadurch gekennzeichnet, dass** die Inkubation zwischen 50 und 60°C durchgeführt wird.

6. Verfahren gemäß einem der Ansprüche 1 bis 5, **dadurch gekennzeichnet, dass** die Inkubation über 2 bis 5 Stunden durchgefährt wird.

7. Verfahren gemäß einem der Ansprüche 1 bis 6, **dadurch gekennzeichnet, dass** die Inkubation in Wasser oder Puffer oder in einem Lösungsmittel-Puffer bzw. Wassergemisch mit einer Puffer-Konzentration in der wässrigen Phase zwischen 0 und 1000 mM und einem pH-Wert zwischen 1 und 13 durchgeführt wird.

8. Verfahren gemäß einem der Ansprüche 1 bis 7, **dadurch gekennzeichnet, dass** die Inkubation mit Endoglucanase-Enzym in einer Konzentration von 0,01 bis 50 % der Cellulose-Masse, durchgeführt wird.

9. Verfahren gemäß einem der Ansprüche 1 bis 8, **dadurch gekennzeichnet, dass** die Inkubation mit Endoglucanase-Enzym in einer Konzentration von 3 bis 15 % der Cellulose-Masse durchgeführt wird.

10. Verfahren gemäß einem der Ansprüche 1 bis 9, **dadurch gekennzeichnet, dass** die Inkubation in Anwesenheit einer geeigneten Konzentration von Bioziden zur Verhinderung des Wachstums von Mikroorganismen und Pilzen durchgeführt wird.

## Claims

1. Process for the preparation of hydroxyalkylcellulose ethers from the reaction of alkene oxides and activated cellulose, **characterised in that** the cellulose is pretreated as follows:
a) incubation in a buffer solution or water or a solvent/buffer or water mixture and endoglucanase,
b) separation of the cellulose pretreated with endoglucanase from the buffer or water or solvent/buffer or water mixture,
c) reaction of the activated cellulose to substituted cellulose derivatives by reaction with alkene oxides in the presence of catalytic alkali.

2. Process according to claim 1, **characterised in that** endoglucanase from fungi, bacteria and plants is used.

3. Process according to claim 2, **characterised in that** there is used endoglucanase from the fungi Trichoderma reesei, Humicola insolens or bacteria of the genera Bacillus, Cellulomonas, Sporocytophaga, Clostridium or Denimax Ultra L® .

4. Process according to any one of claims 1 to 3, **characterised in that** the incubation is carried out at a temperature of from 0°C to 100°C for a period of from 0.1 to 24 hours.

5. Process according to any one of claims 1 to 4, **characterised in that** the incubation is carried out at from 50 to 60°C.

6. Process according to any one of claims 1 to 5, **characterised in that** the incubation is carried out for a period of from 2 to 5 hours.

7. Process according to any one of claims 1 to 6, **characterised in that** the incubation is carried out in water or buffer or in a solvent/buffer or water mixture with a buffer concentration in the aqueous phase of from 0 to 1000 mM and a pH value of from 1 to 13.

8. Process according to any one of claims 1 to 7, **characterised in that** the incubation is carried out with endoglucanase enzyme in a concentration of from 0.01 to 50 % of the weight of the cellulose.

9. Process according to any one of claims 1 to 8, **characterised in that** the incubation is carried out with endoglucanase enzyme in a concentration of from 3 to 15 % of the weight of the cellulose.

10. Process according to any one of claims 1 to 9, **characterised in that** the incubation is carried out in the presence of a suitable concentration of biocides to prevent the growth of microorganisms and fungi.

## Revendications

1. Procédé pour la préparation d'éthers hydroxyalkyliques de la cellulose par réaction d'oxydes d'alkyène avec une cellulose activée, **caractérisé en ce que** la cellulose est soumise au traitement préalable suivant:
a) incubation dans une solution tampon ou dans l'eau ou dans un mélange solvant-tampon ou solvant-tampon-eau et une endoglucanase,
b) séparation de la cellulose traitée au préalable par l'endoglucanase et du mélange tampon ou eau-tampon ou solvant-tampon ou solvant-tampon-eau,
c) conversion de la cellulose activée en dérivés substitués de la cellulose par réaction avec des oxydes d'alkylène en présence d'une quantité catalytique d'alcali.

2. Procédé selon la revendication 1, **caractérisé en ce que** l'on utilise des endoglucanases de mycètes, de bactéries et de végétaux.

3. Procédé selon la revendication 2, **caractérisé en ce que** l'on utilise des endoglucanases des mycètes Trichoderma reesei, Humicola insolens ou de bactéries du genre Bacillus, Cellulomonas, Sporocytophaga, Clostridium ou l'endoglucanase du commerce Denimax Ultra L®.

4. Procédé selon l'une des revendications 1 à 3, **caractérisé en ce que** l'incubation est réalisée à une température de 0 à 100°C en une durée de 0,1 à 24 h.

5. Procédé selon l'une des revendications 1 à 4, **caractérisé en ce que** l'incubation est réalisée à des températures de 50 à 60°C.

6. Procédé selon l'une des revendications 1 à 5, **caractérisé en ce que** l'incubation est réalisée en 2 à 5 h.

7. Procédé selon l'une des revendications 1 à 6, **caractérisé en ce que** l'incubation dans l'eau ou dans un tampon ou dans un mélange solvant-tampon ou solvant-tampon-eau est réalisée à une concentration en tampon de 0 à 1000 mM dans la phase aqueuse et à un pH de 1 à 13.

8. Procédé selon l'une des revendications 1 à 7, **caractérisé en ce que** l'incubation est réalisée avec l'enzyme endoglucanase à une concentration de 0,01 à 50 % de la masse de cellulose.

9. Procédé selon l'une des revendications 1 à 8, **caractérisé en ce que** l'incubation est réalisée avec l'enzyme endoglucanase à une concentration de 3 à 15 % de la masse de cellulose.

10. Procédé selon l'une des revendications 1 à 9, **caractérisé en ce que** l'incubation est réalisée en présence d'une concentration appropriée de biocides empêchant une croissance de micro-organismes et de mycétes.
